# EUROPÄISCHE PATENTANMELDUNG

(11) **EP 0 628 560 A1**
(43) Veröffentlichungstag der Anmeldung: **14.12.1994**
(21) Anmeldenummer: 94108128.3
(22) Anmeldetag: 26.05.1994
(51) Int. Cl.: C07D 495/04, C08G 61/12

(54) **Neue Thiophenderivate**

(30) Priorität: 08.06.1993 DE 4319042
(71) Anmelder: Agfa-Gevaert AG, D-51373 Leverkusen (DE)
(72) Erfinder: Jonas, Friedrich, Dr., D-52066 Aachen (DE)

(57) **Zusammenfassung**

Thiophenderivate der Formel (I) 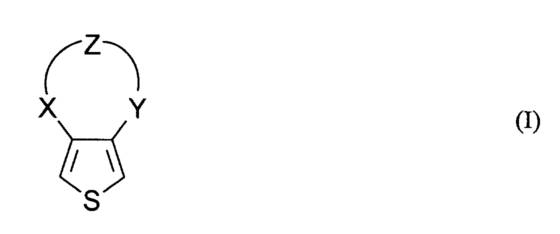
in der
- X und Y: gleich oder verschieden O, S, N-R³,
- Z: einen durch R¹ und R² substituierten C₁-C₆-Alkylenrest,
- R¹, R²: gleich oder verschieden Wasserstoff, -(CH₂)ₙ-OR⁴, 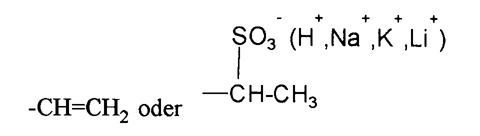
- n: 1 bis 10,
- R³: Aryl, C₁₋₁₈-Alkyl oder H,
- R⁴: SO₃⁻(H⁺, Na⁺, K⁺, Li⁺) oder -(CH₂-CH-O)ₘ-R⁵,
- m: 1 bis 10 und
- R⁵: Alkyl mit 1 bis 6 C-Atomen,
bedeuten,
wobei Verbindungen, in denen X und Y beide O und R¹ und R² beide Wasserstoff bedeuten, ausgeschlossen sind, eignen sich zur Herstellung von Polythiophenen, die als organische elektrische Leiter verwendbar sind.

## Beschreibung

Polythiophene sind als organische elektrische Leiter aus der Literatur bekannt (US 4 987 042).

Ihre Eigenschaften, wie Verarbeitbarkeit oder Langzeitstabilität, sind aber noch nicht für alle technischen Anwendungen ausreichend. Es besteht daher ein Bedarf an Polythiophenen mit verbesserten Eigenschaften.

Die Herstellung der Polythiophene erfolgt durch chemische oder elektrochemische Polymerisation von monomeren oder oligomeren Thiophenen.

Zur Verbesserung der Polythiopheneigenschaften ist es daher erforderlich, neue Thiophenmonomere oder -oligomere zu synthetisieren.

Gegenstand der Erfindung sind zur Herstellung elektrisch leitfähiger Polymere geeignete neue Thiophene der Formel (I) 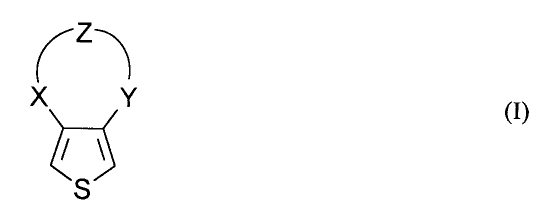
in der
- X und Y: gleich oder verschieden O, S, N-R³,
- Z: einen durch R¹ und R² substituierten C₁-C₆-Alkylenrest,
- R¹, R²: gleich oder verschieden Wasserstoff, oder -(CH₂)ₙ-OR⁴, 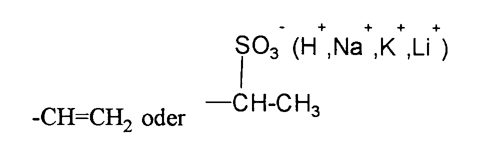
- n: 1 bis 10,
- R³: Aryl, C₁₋₁₈-Alkyl oder H,
- R⁴: SO₃⁻(H⁺, Na⁺, K⁺, Li⁺) oder -(CH₂-CH-O)ₘ-R⁵,
- m: 1 bis 10 und
- R⁵: Alkyl mit 1 bis 6 C-Atomen,
bedeuten,
wobei Verbindungen, in denen X und Y beide O und R¹ und R² beide Wasserstoff bedeuten, ausgeschlossen sind.

Die Herstellung der erfindungsgemäßen Thiophene der Formel (I) erfolgt z.B. auf folgendem Reaktionsweg: 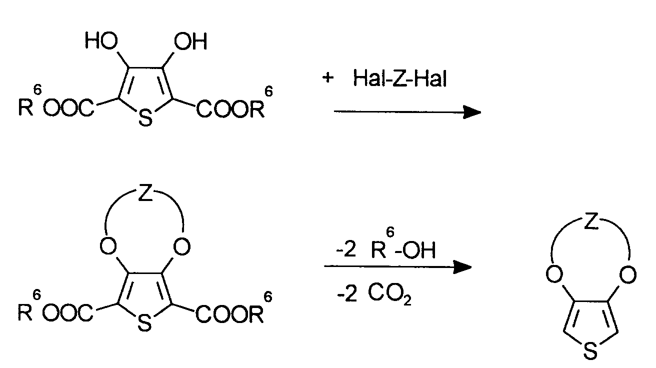
Hierin bedeuten die Reste
- R⁶: Alkylreste mit 1 bis 6 C-Atomen,
- Hal: ein Halogenatom aus der Reihe Fluor, Chlor, Brom oder Iod, während
- Z: die vorstehend genannte Bedeutung hat.

Eine weitere Herstellungsmethode für die erfindungsgemäßen Thiophene der Formel (I) wird durch folgenden Reaktionsweg beschrieben: 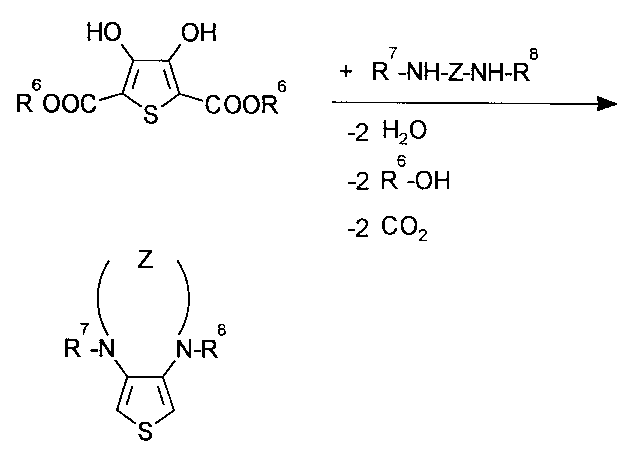
- R⁷ und R⁸: bedeuten Wasserstoff, Aryl oder C₁₋₁₈-Alkyl.
- Z: hat die vorstehend genannte Bedeutung und ist bevorzugt ein nicht weiter substituierter Alkylenrest mit 1 bis 6 C-Atomen.

Die beiden aufgeführten Reaktionswege sind beispielhaft zu verstehen und schließen weitere Herstellungsmethoden für die erfindungsgemäßen Thiophene der Formel (I) nicht aus.

Die Thiophene der Formel sind sehr gut geeignete Ausgangsmaterialien zur Herstellung verarbeitbarer elektrisch leitfähiger Polythiophene.

Die Polymere werden in bekannter Weise durch oxidative Kondensation mit Persulfaten oder Eisen(III)-salzen erhalten und eignen sich zur antistatischen Ausrüstung von Kunststofformkörpern, z.B. von fotografischen Filmen.

### Beispiel

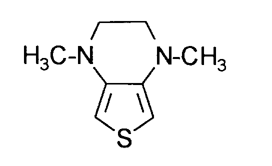

69,6 g 3,4-Dihydroxythiophen-2,5-dicarbonsäuredimethylester, 28,2 g N,N'-Dimethyl-1,2-ethylendiamin, 0,5 g p-Toluolsulfonsäure, 100 ml N,N-Dimethylacetamid und 250 ml Toluol werden 24 Std. bei Rückfluß am Wasserabscheider gerührt. Anschließend wird auf 20.°C abgekühlt und mit 100 ml Toluol verdünnt. Die Lösung wird 4 x mit je 200 ml 5 gew.-%iger Salzsäure extrahiert. Die vereinigten HCl-Phasen werden mit 10 gew.-%iger NaOH alkalisch gestellt und 3 x mit je 150 ml Toluol extrahiert. Die vereinigten Phasen werden fraktioniert destilliert. Kp. = 125-130°C/0,9 mbar; Ausbeute: 8,2 g.

## Patentansprüche

1. Thiophenderivate der Formel (I) 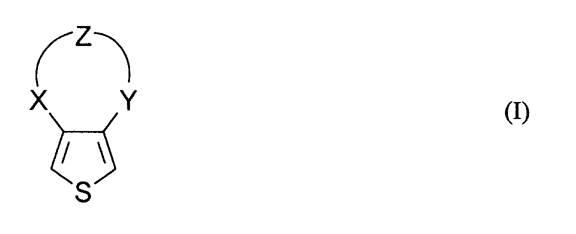 in der
X und Y gleich oder verschieden O, S, N-R³,
Z einen durch R¹ und R² substituierten C₁-C₆-Alkylenrest,
R¹, R² gleich oder verschieden Wasserstoff, -(CH₂)ₙ-OR⁴, 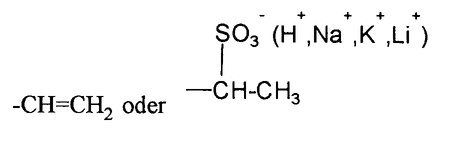
n 1 bis 10,
R³ Aryl, C₁₋₁₈-Alkyl oder H,
R⁴ SO₃⁻(H⁺, Na⁺, K⁺, Li⁺) oder -(CH₂-CH-O)ₘ-R⁵,
m 1 bis 10 und
R⁵ Alkyl mit 1 bis 6 C-Atomen,
bedeuten,
wobei Verbindungen, in denen X und Y beide O und R¹ und R² beide Wasserstoff bedeuten, ausgeschlossen sind.
